# FASCICULE DE BREVET EUROPEEN

(11) **EP 0 674 616 B1**
(45) Date de publication et mention de la délivrance du brevet: **29.07.1998**
(21) Numéro de dépôt: 94902014.3
(22) Date de dépôt: 10.12.1993
(51) Int. Cl.: C07C 231/12, C07C 233/36, A61K 7/075, A61K 7/48

(54) **ACIDES OU SELS D'ACIDES ALPHA-AMINO SUBSTITUES ACETIQUES ET LEUR UTILISATION EN COSMETIQUE**
ALPHA-AMINO-SUBSTITUIERTE ESSIGSÄURE-DERIVATE, IHRE SALZE UND IHRE VERWENDUNG IN DER KOSMETIK
ALPHA-AMINO-SUBSTITUTED ACETIC ACID SALTS OR ACIDS AND COSMETIC USES THEREOF

(30) Priorité: 17.12.1992 FR 9215218
(43) Date de publication de la demande: 04.10.1995
(73) Titulaire: RHODIA CHIMIE, 92408 Courbevoie Cédex (FR)
(72) Inventeur: RICCA, Jean-Marc, F-69007 Lyon (FR)
(74) Mandataire: Fabre, Madeleine-France
(86) Numéro de dépôt international: FR9301223
(87) Numéro de publication internationale: WO9413621

(56) Documents cités:
- EP-A- 0 001 006
- EP-A- 0 373 491

## Description

La présente invention a pour objet des acides ou des sels d'acides α-aminosubstitués acétiques totalement exempts d'acide halogénoacétique et d'halogénure alcalin, ainsi q'un procédé de préparation desdits acides ou sels et l'utilisation desdits acides ou sels d'acides α-aminosubstitués acétiques de très grande pureté comme agents tensio-actifs dans les compositions cosmétiques.

Selon l'invention, il s'agit d'acides ou des sels d'acides α-aminosubstitués acétiques de formule (I) 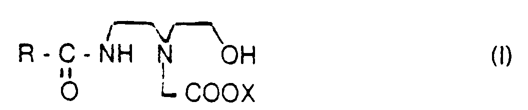 formule dans laquelle
. R représente un atome d'hydrogène ou un groupement alkyle ou alcoxyle, linéaire ou ramifié et éventuellement substitué, en C₁-C₁₈
. X représente un atome d'hydrogène, un métal alcalin ou alcalino-terreux ou reste ammonium
caractérisés en ce qu'ils sont totalement exempts d'acide halogénoacétique et d'halogénure alcalin.

La présente invention vise tout particulièrement la forme acide de la N-lauroyl N' - (hydroxyéthyl-2)-N'- (carboxyméthyl) éthylènediamine et ses sels.

De nombreux composés de formule générale (I) et plus particulièrement leurs sels inorganiques sont largement employés dans les industries de la détergence et de la cosmétique à titre d'agents amphotères. Outre leurs qualités tensio-actives, ces composés s'avèrent également posséder, de part leur structure chimique, un caractère biodégradable très apprécié. A titre de composé particulièrement intéressant, on peut notamment citer le sel de sodium de la N-lauroyl N' - (hydroxyéthyl-2)-N'- (carboxyméthyl) éthylènediamine.

Classiquement, ces composés sont préparés à partir d'un composé de formule générale (II) 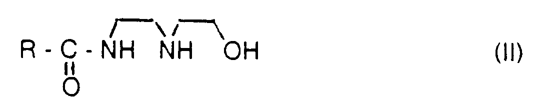 qui subit la condensation de l'acide chloroacétique ou de l'un de ses sels.

Les réactions peuvent être mises en oeuvre en effectuant dans un premier temps une hydrolyse d'une imidazoline de formule : 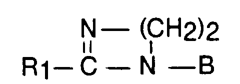 formule dans laquelle R₁ représente un radical aliphatique en C₅-C₂₁, et B est un radical amino-alkyle ou hydroxyalkyle en C₁-C₄ ; avec l'acide chloroacétique ou l'un de ses sels. Un exemple de ce type de procédé est constitué par la demande de brevet européen EP 373 491. Dans ce cas, les produits obtenus correspondent à un mélange comprenant essentiellement un composé de formule : R₁―CO―NH―(CH₂)₂―N(B)(D), formule dans laquelle R₁ et B ont la même signification que précédemment et D représente le radical R₂-COOM où R₂ est un radical alkyle en C₁-C₄ ou un radical alcényle en C₂-C₄, et M représente H, NH₄ ou un métal alcalin.

Une autre voie possible est de mettre en contact directement une imidazoline de formule : 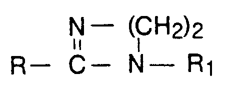 formule dans laquelle R représente un radical aliphatique en C₆-C₂₂, et R₁ est un radical alkyle ou alcényle en C₁-C₄ ; avec l'acide chloroacétique ou l'un de ses sels, selon un procédé décrit notamment dans la demande de brevet européen EP 1 006. Les produits obtenus présentent la formule suivante : R―CO―N(A)―(CH₂)₂―N(B)(C) ; formule dans laquelle A représente le radical R₁ tel que défini auparavant, ou un radical R₂-OO⁻ et R₂ représente un radical alkyle en C₁-C₄, B et C, identiques ou différents représentant un radical R₁ ou R₂-COO⁻.

Ces voies de synthèse ne sont toutefois pas satisfaisantes pour différentes raisons.

La condensation directe d'un acide chloroacétique ne conduit qu'à un faible rendement, de l'ordre de 33 %, et nécessite en outre une étape de purification.

En ce qui concerne plus particulièrement, les sels inorganiques d'acide chloroacétique, ils conduisent certes à de meilleurs rendements mais également à la formation de quantités importantes d'impuretés comme, par exemple, le chlorure de sodium dans le cas particulier du chloroacétate de sodium. Il est alors nécessaire de mettre en oeuvre des techniques lourdes de purification, de type électrodialyse ou osmose qui, outre leur coût, présentent l'inconvénient d'être difficilement industriables.

Enfin, l'acide chloroacétique, de même que ses sels présentent un caractère irritant aujourd'hui bien établi.

Pour ces raisons, il est souhaitable de disposer aujourd'hui de composés de formule (I) totalement exempts d'acide halogénoacétique et d'halogénure alcalin. En outre, les acides ou sels d'acides de l'invention ne contiennent de préférence pas plus de 0,5% de leur poids d'acide glycolique.

Les composés faisant l'objet de l'invention sont susceptibles d'être obtenus par réaction, à chaud, du glyoxal ou d'un précurseur du glyoxal, sur une amine secondaire ou l'un de ses sels de formule générale (II). 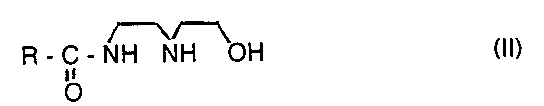 dans laquelle R représente un atome d'hydrogène ou un groupement alkyle ou alcoxyle, linéaire ou ramifié et éventuellement substitué, en C₁-C₁₈, puis éventuellement hydrolyse.

Outre le fait qu'un tel procédé exclue l'emploi de l'acide chloroacétique ou de l'un de ses dérivés, il permet avantageusement un accès direct à la forme acide et s'affranchit donc des contaminants inorganiques classiques comme le chlorure de sodium.

L'amine secondaire de formule générale (II) peut être de préférence obtenue par hydrolyse basique in-situ ou extemporanée dans le milieu réactionnel d'une imidazoline de formule générale (III) 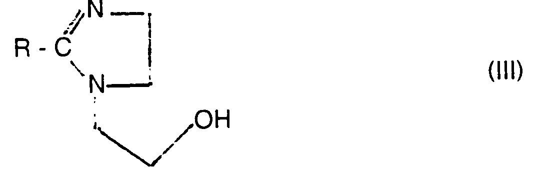 formule dans laquelle R représente un atome d'hydrogène ou un groupement alkyle ou alcoxyle, linéaire ou ramifié et éventuellement substitué, en C₁-C₁₈.

L'hydrolyse de l'imidazoline de formule générale (III) se fait de manière conventionnelle en milieu aqueux et en présence d'une base. Il s'agit plus particulièrement de la soude. L'homme de la technique est à même de part ses compétences techniques à reproduire cette hydrolyse.

Du point de vue des conditions opératoires, ce procédé de préparation des composés de formule (I) est relativement facile de mise en oeuvre.

Quoique la présence d'un excès de glyoxal n'affecte pas le déroulement de la réaction, ce composé est de préférence utilisé en quantité équimolaire par rapport à l'amine secondaire. On s'affranchit ainsi de toute étape de purification subséquente.

Par précurseur du glyoxal, on entend désigner tout composé susceptible de générer du glyoxal dans le milieu réactionnel. Le tétrahydroxy-4,4', 5,5'-bis (dioxolanne-1,3)-2,2 répond notamment à cette définition.

Le glyoxal mis en oeuvre selon l'invention se présente de préférence sous la forme de solutions aqueuses de 30 à 55 % en poids de glyoxal. Bien entendu, il s'avère possible d'employer selon l'invention d'autres formes solubles de glyoxal.

Ces diverses formes sont familières à l'homme de la technique et ne seront donc pas rappelés ici.

A titre de solvant organique susceptible d'être employé selon l'invention on peut notamment citer l'eau, les alcools comme par exemple l'éthanol, le méthanol et les propanols ainsi que leurs mélanges. Il s'agit plus particulièrement de l'eau.

Par réaction à chaud, on entend désigner selon l'invention, une réaction effectuée à une température supérieure ou égale à 60°C.

Avantageusement, il s'agit d'une température comprise entre 60°C et 100°C et de préférence de l'ordre de 80°C.

La durée réactionnelle est fonction de la température. A titre indicatif, une réaction réalisée à 80°C est achevée en environ 2 à 3 heures.

Ce procédé est particulièrement utile pour préparer la forme acide de la N-lauroyl N' - (hydroxyéthyl-2)- N'- (carboxyméthyl) éthylènediamine. Ce composé peut être obtenu par exemple par réaction de condensation, à chaud, du glyoxal sur la N-(hydroxyéthyl-2) laurylimidazoline ou du tétrahydroxy-4,4',5,5'-bis-(dioxolanne-1,3)-2,2' sur la N-lauroyl-N'-(hydroxyéthyl-2) éthylènediamine.

La forme acide des composés de formule générale (I) peut-être convertie ultérieurement en ses sels alcalins, alcalino-terreux ou d'ammonium, par des procédures classiques de neutralisation familières à l'homme de métier et qui ne seront donc pas rappelées ici.

Comme agent de neutralisation susceptibles d'être employés, on peut citer plus spécifiquement les hydroxydes de métaux alcalins, ou alcalino-terreux et les amines comme l'ammoniaque et la triéthanolamine.

Les composés de haute pureté faisant l'objet de l'invention peuvent être mis en oeuvre comme agents tensio-actifs amphotères, pour la confection de compositions cosmétiques telles que shampoings, laits de toilette ...

### Exemple 1

On chauffe, 8 heures à 80°C, un mélange de N-(hydroxyéthyl-2) laurylimidazoline (5g, 18,7 mmol), d'eau (4,4g), de soude en pastilles (40mg) et du glyoxal à 40% dans l'eau (2,7 g, 18,7 mmol). Au bout de 8 heures, un dosage par électrophorèse capillaire indique un rendement de 76 % en N-lauroyl N'-(hydroxyéthyl-2)-N' (carboxyméthyl) éthylènediamine.

### Exemple 2

Une solution de N-(hydroxyéthyl-2) laurylimidazoline (5 g, 18,7mmol) et de soude en pastilles(40 mg) dans l'eau (1,06 g) est chauffée à 80°C pendant 1 heure. Un mélange de glyoxal à 40 % dans l'eau (2,7 g, 18,7 mmol) et de propanol-1 (5 g) est alors ajouté et la solution résiduelle chauffée 6 heures à 80°C. Un dosage par électrophorèse capillaire indique un rendement de 85 % en N-lauroyl-N'-(hydroxyéthyl-2) N'- (carboxyméthyl) éthylènediamine.

### Exemple 3

Un mélange de N-(hydroxyéthyl-2) laurylimidazoline (10 g , 37,3 mmol), d'eau (2 g) et de soude en pastilles (80 mg) est chauffé 1 heure à 80°C. Du glyoxal bis-(sodium hydrogénosulfite) (10,6 g, monohydrate, 37,3 mmol) est alors ajouté et le mélange chauffé 6 heures à 80°C. Un dosage par électrophorèse capillaire indique un rendement de 75 % en N-lauroyl-N'-(hydroxyéthyl-2)-N'-(carboxyméthyl) éthylènediamine.

### Exemple 4

Un mélange de N-lauroyl-N'-(hydroxyéthyl-2) éthylènediamine (2,5 g, 8,74 mmol), de tétrahydroxy-4,4', 5,5'-bis (dioxolanne-1,3)-2,2 cristallisé (0,612 g 2,9 mmol) et d'eau (9,5 g) est chauffé 6 heures à 80°C. Un dosage par électrophorèse capillaire indique un rendement de 97 % en N-lauroyl-N'-(hydroxyéthyl-2)-N'-(carboxyméthyl) éthylénediamine.

### Exemple 5

Un mélange de N-lauroyl-N'-(hydroxyéthyl-2) éthylènediamine (7,28 g , 25,4 mmol) de tétrahydroxy-4,4',5,5'-bis-(dioxolanne-1,3)-2,2' cristallisé (1,783 g, 8,5 mmol), et de propanol-1(10 g) est chauffé 12 heures à 80°C.

Un dosage par électrophorèse capillaire indique un rendement de 19 % en N-lauroyl-N'-(hydroxyéthyl-2)-N'-(carboxyméthyl) éthylènediamine.

### Exemple 6

Un mélange de N-(hydroxyéthyl-2) laurylimidazoline (20 g, 74, 6 mmol), de soude en pastilles (153 mg) et d'eau (4,169 g) est chauffé 1 heure à 80°C. On ajoute alors de l'eau (41,4 g) et on coule en deux heures à 80°C une solution de glyoxal à 21 % dans l'eau (21 g). Le mélange réactionnel est alors chauffé 6 heures à 80°C. Un dosage par électrophorèse capillaire indique un rendement de 97 % en N-lauroyl N'-(hydroxyéthyl-2)-N'-(carboxyméthyl)-éthylènediamine.

Les caractéristiques de profil d'impuretés, pouvoir moussant et pouvoir viscosant de l'agent tensio-actif amphotère préparé à l'exemple 6 sont les suivants

### Impuretés

| pH (solution aqueuse à 10%) | extrait sec (% en poids) | NaCl (% en poids) | glycolate (% en poids) |
|---|---|---|---|
| 8,0 | 30 | 0 | 0,5 |

### Pouvoir moussant

Le pouvoir moussant a été déterminé selon le test Ross-Miles modifié (norme AFNOR T-73 404) à température ambiante, à une concentration de 0,1%.

| volume de mousse (en ml) |
|---|
| 310 |

### Pouvoir viscosant

Celui-ci est mesuré à partir de la formulation suivante

| | |
|---|---|
| - agent tensio-actif amphotère préparé (exemple 6) | |
| - Na lauryl ether sulfate à 3 motifs oxyde d'éthylène (solution aqueuse à 28%) | 35% |
| - eau distillée | 57% |

La concentration en NaCl varie de 0 à 5% ; l'ajout de NaCl augmente la viscosité du milieu.

| extrait sec (%) | viscosité (mPa.s.) | | |
|---|---|---|---|
| | NaCl 0% | NaCl 3% | NaCl 5% |
| 30 | 2 | 51 | 2000 |

### Exemple 7 : Shampooing

Le N-lauroyl-N'-(hydroxyéthyl-2)-N'-(carboxyméthyl) éthylènediamine obtenu selon l'exemple 4 est introduit dans une formulation shampooing comme suit :

| Composants | % matière active | % poids |
|---|---|---|
| Sodium Lauryl Ether Sulfate | 28,3 | 36 |
| N-lauroyl-N'-(hydroxyéthyl-N'carboxyméthyl) éthylènediamine | 50 | 4 |
| Cocoamidopropyl Bétaine | 35 | 4 |
| Polyquaternium-17 | | 0,5 |
| Diéthanolamide de Coprah | | 1,5 |
| Benzoate de Sodium | | 0,2 |
| Colorant | | 0,1 |
| Acide citrique | | QS |
| Chlorure de Sodium | | QS |
| Parfum | | QS |
| Eau distillée | | QSP 100 |
| Cette formulation de shampooing présente un pH de 6,4 et une viscosité de 1678 mPa.s. | | |

L'emploi de la forme acide de la N-lauroyl N'-(hydroxyéthyl-2)-N'-(carboxyméthyl)éthylènediamine permet de imiter considérablement les quantités d'acide citrique nécessaires à l'obtention d'un pH 6,4.

### Exemple 8 : Lait de Toilette pour Bébé

Le composé obtenu selon l'exemple 4 est également introduit dans une formulation de lait de toilette pour bébé. Pour ce faire, on introduit dans de l'eau et sous agitation vigoureuse, de l'hydroxypropyl de Guar, puis on ajuste le pH à 5-6 avec de l'acide citrique. La solution de guar est ensuite introduite dans le mélange de tensio actifs et de N-lauroyl-N'- (hydroxyéthyl-2)-N'-(carboxyméthyl) éthylèndiamine le pH ajusté à 7 avec de l'acide citrique.

| Composants : | % matière active | % Poids |
|---|---|---|
| N-lauroyl-N'-(hydroxyéthyl-2)-N'-(carboxyméthyl) éthylènediamine | 50,0 | 20,0 |
| Hydroxypropyl Guar chlorure de trimonium | | 0,3 |
| Bromo-Nitro-Dioxane | 10,0 | 0,2 |
| Acide citrique | 30,0 | QS |
| Parfum | | 0,2 |
| Colorant | 1,0 | 0,1 |
| Eau distillée | | QSP 100 |
| Le pH est de l'ordre de 7,5 et la viscosité de 10mPa.s | | |

## Revendications

1. Acides ou des sels d'acides α-aminosubstitués acétiques de formule (I) 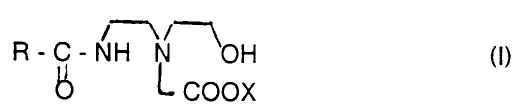 formule dans laquelle
. R représente un atome d'hydrogène ou un groupement alkyle ou alcoxyle, linéaire ou ramifié et éventuellement substitué, en C₁-C₁₈
. X représente un atome d'hydrogène, un métal alcalin ou alcalino-terreux ou reste ammonium
caractérisés en ce qu'ils sont totalement exempts d'acide halogénoacétique et d'halogénure alcalin.

2. Acides ou des sels d'acides α-aminosubstitués acétiques selon la revendication 1) caractérisés en ce qu'ils ne contiennent pas plus de 0,5% de leur poids d'acide glycolique.

3. N'-Lauroyl N - (hydroxéthyl-2)- N'- (carboxyméthyl) éthylènediamine ou ses sels alcalins, alcalino-terreux ou d'ammonium, caractérisé(es) en ce qu'elle (ils) est (sont) totalement exempte (exempts) d'acide halogénoacétique et d'halogénure alcalin.

4. N'-Lauroyl N - (hydroxéthyl-2)- N'- (carboxyméthyl) éthylènediamine ou ses sels alcalins, alcalino-terreux ou d'ammonium, selon la revendication 3) caractérisé(es) en ce qu'elle (ils) ne contient (contiennent) pas plus de 0,5% de son (leur) poids d'acide glycolique.

5. Acides ou des sels d'acides α-aminosubstitués acétiques de formule (I) 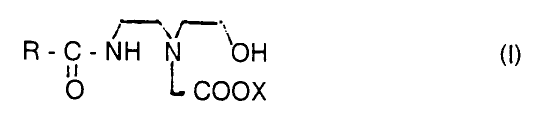 formule dans laquelle
. R représente un atome d'hydrogène ou un groupement alkyle ou alcoxyle, linéaire ou ramifié et éventuellement substitué, en C₁-C₁₈
. X représente un atome d'hydrogène, un métal alcalin ou alcalino-terreux ou reste ammonium
acides ou sels totalement exempts d'acide halogénoacétique et d'halogénure alcalin et caractérisés en ce qu'ils sont susceptibles d'être obtenus par réaction, à chaud, du glyoxal ou d'un précurseur du. glyoxal, sur une amine secondaire ou l'un de ses sels de formule générale (II). 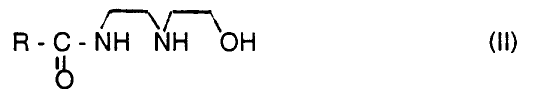 dans laquelle R représente un atome d'hydrogène ou un groupement alkyle ou alcoxyle, linéaire ou ramifié et éventuellement substitué, en C₁-C₁₈, puis éventuellement hydrolyse.

6. Acides ou des sels d'acides selon la revendication 5) caractérisés en ce que l'amine de formule générale (II) est obtenue par hydrolyse basique in-situ ou extemporanée dans le milieu réactionnel d'une imidazoline de formule générale (III) 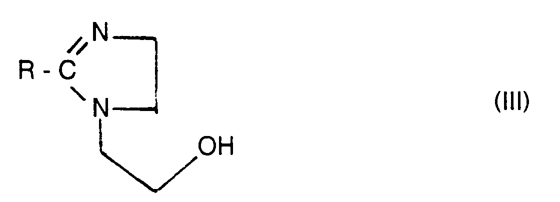 dans laquelle R représente un atome d'hydrogène ou un groupement alkyle ou alcoxyle, linéaire ou ramifié et éventuellement substitué, en C₁-C₁₈.

7. Acides ou des sels d'acides selon la revendication 5) ou 6) caractérisés en ce que le glyoxal ou précurseur de glyoxal et l'amine secondaire sont mis en présence dans un rapport molaire égal à 1.

8. Acides ou des sels d'acides selon l'une quelconque des revendications 5) à 7) caractérisés en ce que la réaction de condensation est réalisée à une température supérieure ou égale à 60°C.

9. Acides ou des sels d'acides selon l'une quelconque des revendications 5) à 8) caractérisés en ce que la température est de préférence comprise entre 60 et 100°C et est préférentiellement de l'ordre de 80°C.

10. Acides ou des sels d'acides selon l'une quelconque des revendications 5) à 9) caractérisés en ce que la réaction est effectuée en présence d'eau.

11. N'-Lauroyl N - (hydroxéthyl-2)- N'- (carboxyméthyl) éthylènediamine ou ses sels alcalins, alcalino-terreux ou d'ammonium, caractérisé(es) en ce qu'elle (ils) est (sont) susceptible(s) d'être obtenue (obtenus) par réaction de condensation, à chaud, du glyoxal sur la N-(hydroxyéthyl-2) laurylimidazoline ou du tétrahydroxy-4,4',5,5'-bis-(dioxolanne-1,3)-2,2' sur la N-lauroyl-N'-(hydroxyéthyl-2) éthylènediamine.

12. N'-Lauroyl N - (hydroxéthyl-2)- N'- (carboxyméthyl) éthylènediamine ou ses sels alcalins, alcalino-terreux ou d'ammonium, selon la revendication 11) caractérisée(és) en ce que les réactifs sont mis en présence dans un rapport molaire égal à 1.

13. N'-Lauroyl N - (hydroxéthyl-2)- N'- (carboxyméthyl) éthylènediamine ou ses sels alcalins, alcalino-terreux ou d'ammonium, selon l'une des revendications 11) ou 12) caractérisée(és) en ce que la réaction de condensation est réalisée a une température supérieure ou égale à 60°C.

14. N'-Lauroyl N - (hydroxéthyl-2)- N'- (carboxyméthyl) éthylènediamine ou ses sels alcalins, alcalino-terreux ou d'ammonium, selon l'une quelconque des revendications 11) à 13) caractérisée(és) en ce que la température est de préférence comprise entre 60 et 100°C et est préférentiellement de l'ordre de 80°C.

15. N'-Lauroyl N - (hydroxéthyl-2)- N'- (carboxyméthyl) éthylènediamine ou ses sels alcalins, alcalino-terreux ou d'ammonium, selon l'une quelconque des revendications 11) à 14) caractérisée(és) en ce que la réaction de condensation est effectuée en présence d'eau.

16. Procédé de préparation d'acides ou des sels d'acides α-aminosubstitués acétiques de formule (I) 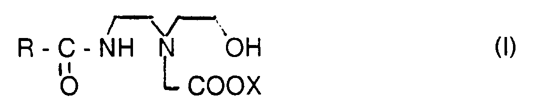 formule dans laquelle
. R représente un atome d'hydrogène ou un groupement alkyle ou alcoxyle, linéaire ou ramifié et éventuellement substitué, en C₁-C₁₈
. X représente un atome d'hydrogène, un métal alcalin ou alcalino-terreux ou reste ammonium
caractérisé en ce qu'on fait réagir par condensation , à chaud, du glyoxal ou un précurseur du glyoxal, sur une amine secondaire ou l'un de ses sels de formule générale (II). 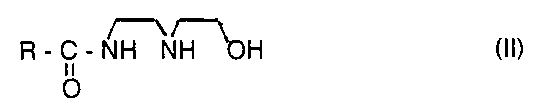 dans laquelle R représente un atome d'hydrogène ou un groupement alkyle ou alcoxyle, linéaire ou ramifié et éventuellement substitué, en C₁-C₁₈, puis éventuellement hydrolyse.

17. Procédé selon la revendication 16) caractérisé en ce que l'amine de formule générale (II) est obtenue par hydrolyse basique in-situ ou extemporanée dans le milieu réactionnel d'une imidazoline de formule générale (III) 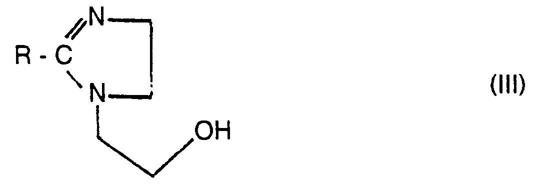 dans laquelle R représente un atome d'hydrogène ou un groupement alkyle ou alcoxyle, linéaire ou ramifié et éventuellement substitué, en C₁-C₁₈.

18. Procédé de préparation de N'-Lauroyl N - (hydroxéthyl-2)- N'-(carboxyméthyl) éthylènediamine ou ses sels alcalins, alcalino-terreux ou d'ammonium, par réaction de condensation, à chaud, du glyoxal sur la N-(hydroxyéthyl-2) laurylimidazoline ou du tétrahydroxy-4,4',5,5'-bis-(dioxolanne-1,3)-2,2' sur la N-lauroyl-N'-(hydroxyéthyl-2) éthylènediamine.

19. Procédé selon la revendication 16) ou 18) caractérisé en ce que le glyoxal ou précurseur de glyoxal et l'amine secondaire sont mis en présence dans un rapport molaire égal à 1.

20. Procédé selon l'une quelconque des revendications 16) à 19) caractérisé en ce que la réaction de condensation est réalisée à une température supérieure ou égale à 60°C.

21. Procédé selon l'une quelconque des revendications 16) à 20) caractérisé en ce que la température est de préférence comprise entre 60 et 100°C et est préférentiellement de l'ordre de 80°C.

22. Procédé selon l'une quelconque des revendications 16) à 21) caractérisé en ce que la réaction est effectuée en présence d'eau.

23. Utilisation des acides ou des sels d'acides α-aminosubstitués acétiques faisant l'objet de l'une quelconque des revendications 1) à 15) ou obtenus selon le procédé de l'une quelconque des revendications 16) à 22) comme agents tensio-actifs amphotères dans les compositions cosmétiques.

## Claims

1. α-Amino-substituted acetic acids or acid salts of formula (I) 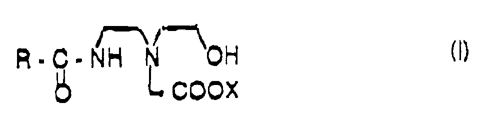 in which formula
• R represents a hydrogen atom or a linear or branched and optionally substituted C₁-C₁₈ alkyl or alkoxy group,
• X represents a hydrogen atom, an alkali metal or alkaline-earth metal or an ammonium residue
characterized in that they are totally free of haloacetic acid and alkali metal halide.

2. α-Amino-substituted acetic acids or acid salts according to Claim 1, characterized in that they contain no more than 0.5% of their weight of glycolic acid.

3. N-Lauroyl-N'-(2-hydroxyethyl)-N'-(carboxymethyl)ethylenediamine or its salts with alkali metals, alkaline-earth metals or ammonium, characterized in that it (they) is (are) totally free of haloacetic acid and alkali metal halide.

4. N-Lauroyl-N'-(2-hydroxyethyl)-N'-(carboxymethyl)ethylenediamine or its salts with alkali metals, alkaline-earth metals or ammonium, according to Claim 3, characterized in that it (they) contain no more than 0.5% of its (their) weight of glycolic acid.

5. α-Amino-substituted acetic acids or acid salts of formula (I) 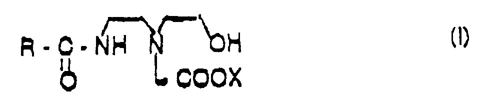 in which formula
• R represents a hydrogen atom or a linear or branched and optionally substituted C₁-C₁₈ alkyl or alkoxy group,
• X represents a hydrogen atom, an alkali metal or alkaline-earth metal or an ammonium residue,
which acids or salts are totally free of haloacetic acid and alkali metal halide and are characterized in that they are capable of being obtained by reaction, under hot conditions, of glyoxal or of a precursor of glyoxal, with a secondary amine or one of its salts of general formula (II), 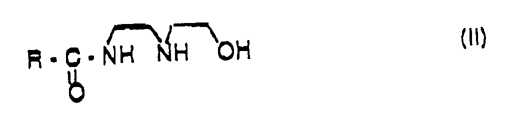 in which R represents a hydrogen atom or a linear or branched and optionally substituted C₁-C₁₈ alkyl or alkoxy group, optionally followed by hydrolysis.

6. Acids or acid salts according to Claim 5, characterized in that the amine of general formula (II) is obtained by in situ or extemporaneous basic hydrolysis, in the reaction medium, of an imidazoline of general formula (III) 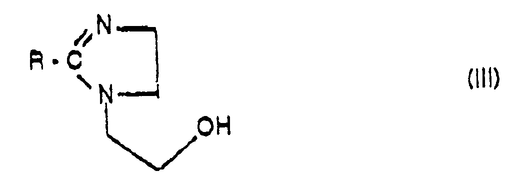 in which R represents a hydrogen atom or a linear or branched and optionally substituted C₁-C₁₈ alkyl or alkoxy group.

7. Acids or acid salts according to Claim 5 or 6, characterized in that glyoxal or the precursor of glyoxal and the secondary amine are brought together in a molar ratio equal to 1.

8. Acids or acid salts according to any one of Claims 5 to 7, characterized in that the condensation reaction is carried out at a temperature greater than or equal to 60°C.

9. Acids or acid salts according to any one of Claims 5 to 8, characterized in that the temperature is preferably between 60 and 100°C, and is preferably of the order of 80°C.

10. Acids or acid salts according to any one of Claims 5 to 9, characterized in that the reaction is carried out in the presence of water.

11. N-Lauroyl-N'-(2-hydroxyethyl)-N'-(carboxymethyl)ethylenediamine or its salts with alkali metals, alkaline-earth metals or ammonium, characterized in that it (they) is (are) capable of being obtained by the condensation reaction, under hot conditions, of glyoxal with N-(2-hydroxyethyl)lauryl-imidazoline or 4,4',5,5'-tetrahydroxybis[2,2-(1,3-dioxolane)] with N-lauroyl-N'-(2-hydroxyethyl)-ethylenediamine.

12. N-Lauroyl-N'-(2-hydroxyethyl)-N'-(carboxymethyl)ethylenediamine or its salts with alkali metals, alkaline-earth metals or ammonium, according to Claim 11, characterized in that the reactants are brought together in a molar ratio equal to 1.

13. N-Lauroyl-N'-(2-hydroxyethyl)-N'-(carboxymethyl)ethylenediamine or its salts with alkali metals, alkaline-earth metals or ammonium, according to either of Claims 11 and 12, characterized in that the condensation reaction is carried out at a temperature greater than or equal to 60°C.

14. N-Lauroyl-N'-(2-hydroxyethyl)-N'-(carboxymethyl)ethylenediamine or its salts with alkali metals, alkaline-earth metals or ammonium, according to any one of Claims 11 to 13, characterized in that the temperature is preferably between 60°C and 100°C and is preferably of the order of 80°C.

15. N-Lauroyl-N'-(2-hydroxyethyl)-N'-(carboxymethyl)ethylenediamine or its salts with alkali metals, alkaline-earth metals or ammonium, according to any one of Claims 11 to 14, characterized in that the condensation reaction is carried out in the presence of water.

16. Process for the preparation of α-amino-substituted acetic acids or acid salts of formula (I) 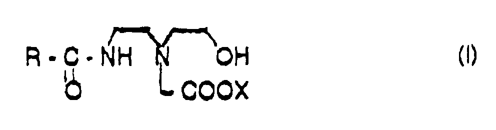 in which formula
• R represents a hydrogen atom or a linear or branched and optionally substituted C₁-C₁₈ alkyl or alkoxy group
• X represents a hydrogen atom, an alkali metal or alkaline-earth metal or an ammonium residue
characterized in that glyoxal or a precursor of glyoxal is reacted by condensation, under hot conditions, with a secondary amine or with one of its salts of general formula (II), 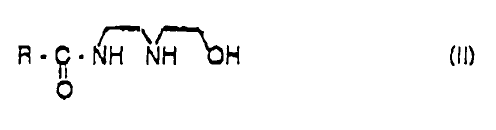 in which R represents a hydrogen atom or a linear or branched and optionally substituted C₁-C₁₈ alkyl or alkoxy group, optionally followed by hydrolysis.

17. Process according to Claim 16, characterized in that the amine of general formula (II) is obtained by in situ or extemporaneous basic hydrolysis, in the reaction medium, of an imidazoline of general formula (III) 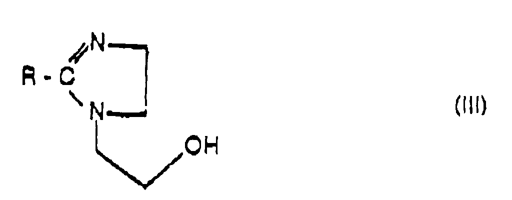 in which R represents a hydrogen atom or a linear or branched and optionally substituted C₁-C₁₈ alkyl or alkoxy group.

18. Process for the preparation of N-lauroyl-N'-(2-hydroxyethyl)-N'-(carboxymethyl)-ethylenediamine or its salts with alkali metals, alkaline-earth metals or ammonium, by the condensation reaction, under hot conditions, of glyoxal with N-(2-hydroxyethyl)lauryl-imidazoline or 4,4',5,5'-tetrahydroxybis[2,2-(1,3-dioxolane)] with N-lauroyl-N'-(2-hydroxyethyl)ethylenediamine.

19. Process according to Claim 16 or 18, characterized in that glyoxal or the precursor of glyoxal and the secondary amine are placed together in a molar ratio equal to 1.

20. Process according to any one of Claims 16 to 19, characterized in that the condensation reaction is carried out at a temperature greater than or equal to 60°C.

21. Process according to any one of Claims 16 to 20, characterized in that the temperature is preferably between 60 and 100°C, and is preferably of the order of 80°C.

22. Process according to any one of Claims 16 to 21, characterized in that the reaction is carried out in the presence of water.

23. Use of the α-amino-substituted acetic acids or acid salts forming the subject of any one of Claims 1 to 15 or which are obtained according to the process of any one of Claims 16 to 22 as amphoteric surface-active agents in cosmetic compositions.

## Patentansprüche

1. α-aminosubstituierte Essigsäuren beziehungsweise deren Salze der Formel (I): 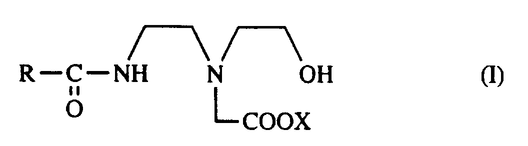 in der:
- R ein Wasserstoffatom oder eine geradkettige oder verzweigte und gegebenenfalls substituierte C₁-C₁₈-Alkyl- oder Alkoxylgruppe darstellt und
- X ein Wasserstoffatom, ein Alkali- oder Erdalkalimetall oder einen Ammoniumrest darstellt,
dadurch gekennzeichnet, daß sie vollkommen frei von Halogenessigsäuren und von Alkalimetallhalogeniden sind.

2. α-aminosubstituierte Essigsäuren beziehungsweise deren Salze nach Anspruch 1, dadurch gekennzeichnet, daß sie, bezogen auf ihr Eigengewicht, nicht mehr als 0,5% Glykolsäure enthalten.

3. N'-Lauroyl-N-(2-hydroxyethyl)-N'-carboxymethyl-ethylendiamin beziehungsweise dessen Alkalimetall-, Erdalkalimetall- oder Ammoniumsalze, dadurch gekennzeichnet, daß sie vollkommen frei von Halogenessigsäuren und von Alkalimetallhalogeniden sind.

4. N'-Lauroyl-N-(2-hydroxyethyl)-N'-carboxymethyl-ethylendiamin beziehungsweise dessen Alkalimetall-, Erdalkalimetall- oder Ammoniumsalze nach Anspruch 3, dadurch gekennzeichnet, daß sie, bezogen auf ihr Eigengewicht, nicht mehr als 0,5% Glykolsäure enthalten.

5. α-aminosubstituierte Essigsäuren beziehungsweise deren Salze der Formel (I): 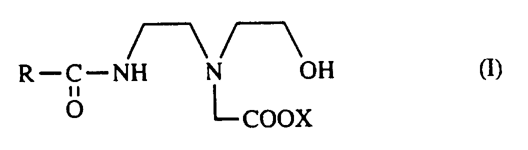 in der:
- R ein Wasserstoffatom oder eine geradkettige oder verzweigte und gegebenenfalls substituierte C₁-C₁₈-Alkyl- oder Alkoxylgruppe darstellt und
- X ein Wasserstoffatom, ein Alkali- oder Erdalkalimetall oder einen Ammoniumrest darstellt,
wobei diese Säuren beziehungsweise Salze vollkommen frei von Halogenessigsäuren und von Alkalimetallhalogeniden und dadurch gekennzeichnet sind, daß sie durch Umsetzung von Glyoxal oder einer Glyoxal-Vorstufe mit einem sekundären Amin oder einem der Salze der allgemeinen Formel (II): 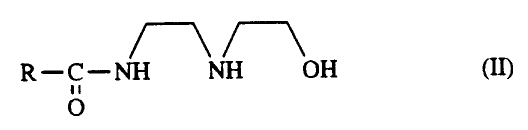 in der R ein Wasserstoffatom oder eine geradkettige oder verzweigte und gegebenenfalls substituierte C₁-C₁₈-Alkyl- oder Alkoxylgruppe darstellt,
in der Hitze und gegebenenfalls anschließende Hydrolyse erhalten werden können.

6. Säuren beziehungsweise Salze von Säuren nach Anspruch 5, dadurch gekennzeichnet, daß das Amin der allgemeinen Formel (II) in situ beziehungsweise während der Reaktion im Reaktionsmedium durch basische Hydrolyse eines Imidazolins der folgenden allgemeinen Formel (III) erhalten wird: 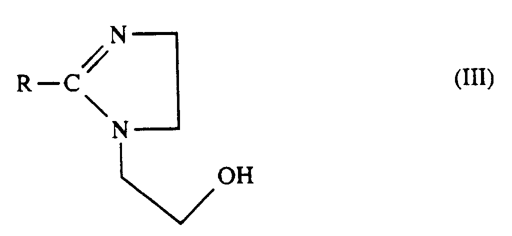 in der:
R ein Wasserstoffatom oder eine geradkettige oder verzweigte und gegebenenfalls substituierte C₁-C₁₈-Alkyl- oder Alkoxylgruppe darstellt.

7. Säuren beziehungsweise Salze von Säuren nach Anspruch 5 oder 6, dadurch gekennzeichnet, daß das Glyoxal beziehungsweise die Glyoxal-Vorstufe und das sekundäre Amin in einem molaren Verhältnis von 1:1 miteinander umgesetzt werden.

8. Säuren beziehungsweise Salze von Säuren nach einem der Ansprüche 5 bis 7, dadurch gekennzeichnet, daß die Kondensationsreaktion bei einer Temperatur größer oder gleich 60 °C durchgeführt wird.

9. Säuren beziehungsweise Salze von Säuren nach einem der Ansprüche 5 bis 8, dadurch gekennzeichnet, daß die Temperatur vorzugsweise im Bereich von 60 bis 100 °C und im besonderen in der Größenordnung von 80 °C liegt.

10. Säuren beziehungsweise Salze von Säuren nach einem der Ansprüche 5 bis 9, dadurch gekennzeichnet, daß die Reaktion in Gegenwart von Wasser durchgeführt wird.

11. N'-Lauroyl-N-(2-hydroxyethyl)-N'-carboxymethyl-ethylendiamin beziehungsweise dessen Alkalimetall-, Erdalkalimetall- oder Anunoniumsalze, dadurch gekennzeichnet, daß sie durch eine Kondensationsreaktion von Glyoxal mit N-(2-Hydroxyethyl)-laurylimidazolin oder von 4,4',5,5'-Tetrahydroxy-2,2'-bis-(1,3-dioxolan) mit N-Lauroyl-N'-(2-hydroxyethyl)-ethylendiamin in der Hitze erhalten werden können.

12. N'-Lauroyl-N-(2-hydroxyethyl)-N'-carboxymethyl-ethylendiamin beziehungsweise dessen Alkalimetall-, Erdalkalimetall- oder Ammoniumsalze nach Anspruch 11, dadurch gekennzeichnet, daß die Reaktanten in einem molaren Verhältnis von 1:1 miteinander umgesetzt werden.

13. N'-Lauroyl-N-(2-hydroxyethyl)-N'-carboxymethyl-ethylendiamin beziehungsweise dessen Alkalimetall-, Erdalkalimetall- oder Ammoniumsalze nach Anspruch 11 oder 12, dadurch gekennzeichnet, daß die Kondensationsreaktion bei einer Temperatur größer oder gleich 60 °C durchgeführt wird.

14. N'-Lauroyl-N-(2-hydroxyethyl)-N'-carboxymethyl-ethylendiamin beziehungsweise dessen Alkalimetall-, Erdalkalimetall- oder Ammnoniumsalze nach einem der Ansprüche 11 bis 13, dadurch gekennzeichnet, daß die Temperatur vorzugsweise im Bereich von 60 bis 100 °C und im besonderen in der Größenordnung von 80 °C liegt.

15. N'-Lauroyl-N-(2-hydroxyethyl)-N'-carboxymethyl-ethylendiamin beziehungsweise dessen Alkalimetall-, Erdalkalimetall- oder Ammoniumsalze nach einem der Ansprüche 11 bis 14, dadurch gekennzeichnet, daß die Kondensationsreaktion in Gegenwart von Wasser durchgeführt wird.

16. Verfahren zur Herstellung von α-aminosubstituierten Essigsäuren beziehungsweise deren Salzen der Formel (I): 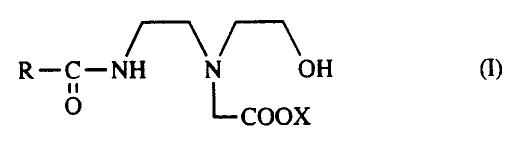 in der:
- R ein Wasserstoffatom oder eine geradkettige oder verzweigte und gegebenenfalls substituierte C₁-C₁₈-Alkyl- oder Alkoxylgruppe darstellt und
- X ein Wasserstoffatom, ein Alkali- oder Erdalkalimetall oder einen Ammnoniumrest darstellt,
dadurch gekennzeichnet, daß in der Hitze eine Kondensationsreaktion von Glyoxal oder einer Glyoxal-Vorstufe mit einem sekundären Amin oder einem der Salze der allgemeinen Formel (II): 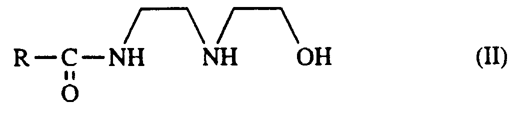 in der R ein Wasserstoffatom oder eine geradkettige oder verzweigte und gegebenenfalls substituierte C₁-C₁₈-Alkyl- oder Alkoxylgruppe darstellt,
und gegebenenfalls anschließend eine Hydrolyse durchgeführt wird.

17. Verfahren nach Anspruch 16, dadurch gekennzeichnet, daß das Amin der allgemeinen Formel (II) in situ beziehungsweise während der Reaktion im Reaktionsmedium durch basische Hydrolyse eines Imidazolins der folgenden allgemeinen Formel (III) erhalten wird: 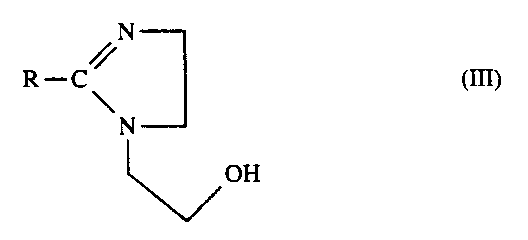 in der:
R ein Wasserstoffatom oder eine geradkettige oder verzweigte und gegebenenfalls substituierte C₁-C₁₈-Alkyl- oder Alkoxylgruppe darstellt.

18. Verfahren zur Herstellung von N'-Lauroyl-N-(2-hydroxyethyl)-N'-carboxymethyl ethylendiamin beziehungsweise dessen Alkalimetall-, Erdalkalimetall- oder Alnmoniumsalzen durch eine Kondensationsreaktion von Glyoxal mit N-(2-Hydroxyethyl)-laurylimidazolin oder von 4,4',5,5'-Tetrahydroxy-2,2'-bis-(1,3-dioxolan) mit N-Lauroyl-N'-(2-hydroxyethyl)-ethylendiamin in der Hitze.

19. Verfahren nach Anspruch 16 oder 18, dadurch gekennzeichnet, daß das Glyoxal beziehungsweise die Glyoxal-Vorstufe und das sekundäre Amin in einem molaren Verhältnis von 1:1 miteinander umgesetzt werden.

20. Verfahren nach einem der Ansprüche 16 bis 19, dadurch gekennzeichnet, daß die Kondensationsreaktion bei einer Temperatur größer oder gleich 60 °C durchgeführt wird.

21. Verfahren nach einem der Ansprüche 16 bis 20, dadurch gekennzeichnet, daß die Temperatur vorzugsweise im Bereich von 60 bis 100 °C und im besonderen in der Größenordnung von 80 °C liegt.

22. Verfahren nach einem der Ansprüche 16 bis 21, dadurch gekennzeichnet, daß die Reaktion in Gegenwart von Wasser durchgeführt wird.

23. Verwendung von α-aminosubstituierten Essigsäuren beziehungsweise deren Salzen, die Gegenstand eines der Ansprüche 1 bis 15 sind oder die nach dem Verfahren nach einem der Ansprüche 16 bis 22 hergestellt werden, als amphotere grenzflächenaktive Mittel in kosmetischen Zusammensetzungen.
